# EUROPEAN PATENT APPLICATION

(11) **EP 1 982 719 A1**
(43) Date of publication of application: **22.10.2008**
(21) Application number: 08075587.9
(22) Date of filing: 24.03.2003
(51) Int. Cl.: A61K 31/585, A61K 31/565, A61K 31/567, A61K 31/401, A61K 38/55, A61K 31/675, A61P 9/12, A61P 15/12

(54) **Treatment of hypertension in women receiving hormone replacement therapy**

(30) Priority: 26.04.2002 US 375439 P
(62) Divisional of application: 03733879.5
(71) Applicant: Bayer Schering Pharma Aktiengesellschaft, 13353 Berlin (DE)
(72) Inventor: Karara, Adel H., Branchburg, NJ 08876 (US)

(57) **Abstract**

A method is described to lower blood pressure and effect hormone replacement therapy (HRT) in a hypertensive woman receiving HRT, comprising administering to the woman effective amounts of drospirenone and an estrogen. In one embodiment, the woman is already taking hypertensive medicine when the method is carried out.

## Description

This application claims the benefit of U.S. Provisional Application Serial No. 60/375,439, filed April 26, 2002, which is incorporated herein in full by reference.

### DESCRIPTION OF THE INVENTION

The present invention is directed *e.g*., to a method to lower blood pressure and effect hormone replacement therapy (HRT) in a hypertensive human female (woman) receiving HRT, comprising administering to the woman an effective amount of drospirenone and an estrogen. In one embodiment, the woman simultaneously takes antihypertensive medicine.

Drospirenone (DRSP) is a 17-α-spirolactone derivative progestin that, in combination with estrogens, is used for hormone replacement therapy (HRT), *e.g*., in postmenopausal women. The present inventor has found, unexpectedly, that when hypertensive women in need of HRT, *e.g*., women who are already taking antihypertensive medication, are administered a combination of DRSP and estrogen, their blood pressure is unexpectedly reduced compared to women taking the antihypertensive alone.

In one embodiment of the invention, the estrogen is ethinyl estradiol, 17β-estradiol or an ester thereof, or a conjugated estrogen; the composition is administered orally, transdermally or by injection, preferably orally; the estrogen is 17β-estradiol, at about 1-3 mg/day, and the drospirenone is at about 1-3 mg/day; the woman is postmenopausal; and/or the hypertension is mild hypertension or severe hypertension.

In one embodiment, a woman treated by the method of the invention is not taking hypertensive medication. See, e.g., Example 3 herein.

In another embodiment, the woman to whom the inventive combination is administered also takes antihypertensive medication, beginning before or after the combination is administered, or beginning in conjunction with the administration of said combination. The antihypertensive medicine can be, *e.g*., a diuretic, an α-adrenergic blocking agent, an adrenergic neuron blocking agent, a vasodilator, an angiotensin I converting enzyme (ACE) inhibitor, a calcium channel blocker, or a renin inhibitor, typically an ACE inhibitor.

In another embodiment of the invention, the blood pressure of hypertensive women administered the combination of drospirenone and estradiol without an antihypertensive medication was also unexpectedly reduced, see Example 3.

A woman suitable for treatment by the method of the invention is hypertensive (suffering from hypertension, having elevated blood pressure). She may suffer from any degree of hypertension, *e.g*. borderline, mild (*e.g*., stage 1), or severe hypertension.

The blood pressure that is lowered can be diastolic, systolic, mean and/or arterial blood pressure, or combinations thereof, and can be ambulatory blood pressure or office blood pressure (preferably ambulatory), measured at rest or during exercise, during the day and/or at night, and/or at any desirable time after administration of the combination of the invention. Typically, blood pressure is lowered within about 14 days after the combination is administered.

Blood pressure can be measured by any of a variety of conventional procedures. See, for example, the Examples herein for some methods of monitoring blood pressure in human subjects.

A woman suitable for treatment by the method of the invention is in need of hormone replacement therapy (HRT). Typically, the woman is menopausal or postmenopausal. As used herein, the terms "menopause" and "menopausal" include both the perimenopausal and postmenopausal states. Women in need of HRT include women who, as a result of the normal aging process, have entered or begun to enter menopause, or women who have ceased menstruation for non-age-related reasons, *e.g.*, due to excessive exercise or as a result of surgery (*e.g*., hysterectomy, oophorectomy).

In one embodiment, a woman treated by the method of the invention is taking antihypertensive medication, *e.g*., she is already taking an antihypertensive medication before the combination of the invention is administered, or she begins taking an antihypertensive medication concomitantly with, or after, administration of the combination of the invention. The interval of time between the start of the antihypertensive medication treatment and the administration of the combination of the invention is not critical. In a preferred embodiment, the decrease in blood pressure in a woman receiving a combination of the invention is greater than the decrease achieved when the anti-hypertensive medication, alone, is administered.

Women treated by the method of the invention can be taking any of a variety of conventional antihypertensive medications (medications that are effective in reducing or alleviating hypertension), including, *e.g*., diuretics, α- and/or β-adrenergic blocking agents, CNS-acting agents, adrenergic neuron blocking agents, vasodilators, angiotensin I converting enzyme (ACE) inhibitors, calcium channel blockers, renin inhibitors, or other antihypertensive agents, or combinations thereof.

Diuretics include, *e.g*., acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, *e.g*., 9:1 of said enantiomers, respectively); metolazone; methyclothiazide; muzolimine; polythiazide; quinethazone; sodium ethacrynate; sodium nitroprusside; ticrynafen; triamterene; and trichlormethiazide.
α -adrenergic blocking agents include, *e.g*., dibenamine; phentolamine; phenoxybenzamine; prazosin; and tolazoline.
β -adrenergic blocking agents include, *e.g*., atenolol; metoprolol; nadolol; propranolol; timolol; and various other agents listed in USP 5,001,113.

CNS-acting agents include, *e.g*., clonidine and methyldopa.

Adrenergic neuron blocking agents include, e.g., quanethidine; reserpine and other rauwolfia alkaloids such as rescinnamine.

Vasodilators include, *e.g*., diazoxide; hydralazine; and minoxidil.

Calcium channel blockers include, *e.g*., those listed in USP 5,001,113.

Renin-inhibitors include, *e.g*., pepstatin and the di- or tripeptide renin inhibitors disclosed is USP 5,001,113 and in references therein.

Other antihypertensive agents include, *e.g*., aminophylline; cryptenamine acetates and tannates; deserpidine; meremethoxylline procaine; pargyline; and tirmethaphan camsylate.

In a typical case, a woman being treated with the method of the invention is taking an ACE inhibitor, such as, *e.g.*, benazepril, captopril, enalapril, enalaprilat, fosinopril, lisinopril, pentopril, quinapril, quinaprilat, ramipril, transolapril, zofenopril; peptides such as those described in USP 6,232,438 and in references cited therein; compounds as described in USP 6,300,362; agents such as those listed in USP 5,001,113; and the like.

Such antihypertensive agents are administered according to conventional procedures, using conventional dosages, kits, regimens of administration, and modes of administration.

Any of a variety of estrogens, as is well known in the art, can be used in methods of hormone replacement therapy. Such estrogens include, *e.g*., ethinyl estradiol, mestranol, estradiol (especially 17-β-estradiol, known as E2) and esters thereof (*e.g*., valerate, acetate, benzoate or undecylate); estriol; estriol succinate; polyestriol phosphate; estrone; estrone sulfate; natural or synthetic estrogens; and conjugated estrogens.

DRSP can be obtained from commercial sources (*e.g.*, from Schering Aktiengesellschaft ) or can by synthesized by conventional methods, *e.g*., according to the methods disclosed in USP 6,121,465 and Drugs of the Future 2000 25 (12), 1247-1256.

DRSP and an estrogen can be administered to a patient following conventional procedures, using conventional regimens of administration, kits, modes of administration, and dosages, all of which are well known to those of skill in the art.

Regimens are conventional and well known in the art for HRT purposes. The estrogen and DRSP can be administered concurrently, for any period of time, *e.g*., on a daily basis, 1-4 times a week, weekly, 2-3 weeks per month, etc. The two components can be administered separately (as disclosed, *e.g*., in USP 6,083,528), e.g., via a conventional kit, or as a combined preparation (*e.g*., a tablet or capsule).

The pharmaceutical compositions of the invention can be administered by any of a variety of conventional modes, including, *e.g*., oral (*e.g*., solutions, suspensions, tablets, dragees, capsules or pills), parenteral (including subcutaneous injection, or intravenous, intramuscular or intrastemal injection or infusion techniques), inhalation spray, transdermal, rectal, or vaginal (*e.g*., by vaginal rings or creams) administration. The two components can be administered by the same mode, or by different modes (*e.g*., transdermal estrogen and intravaginal DRSP).

A dosage that is "effective" to effect hormone replacement therapy is one that prevents or diminishes (alleviates) adverse physiological effects or symptoms resulting from reduced amounts of estrogen, such as, *e.g*., bone loss and resultant structural deformation, among many others. A dosage of a composition of the invention that is "effective" to reduce blood pressure is one that can achieve a measurable decrease in blood pressure. Any effective dosage can be administered in the methods of the invention, preferably a low dose formulation.

Effective dosages of estrogens are conventional and well known in the art. Typical approximate dosages for oral administration are, *e.g*., ethinyl estradiol (0.001-0.030 mg/day), mestranol (5-25 mcg/day), estradiol (including 17-β estradiol), (0.5-6 mg/day), polyestriol phosphate (2-8 mg) and conjugated estrogens (0.3-1.2 mg/day). Dosages for other means of delivery will be evident to one of skill in the art. For example, transdermal dosages will vary therefrom in accordance with the adsorption efficacy of the vehicle employed.

Effective dosages of drospirenone are also conventional and well known in the art. Typical dosages for oral administration are about 1.0-3.0 mg/day.

Preferred combinations of the invention include, for oral administration, 3 mg DRSP/1 mg E2 and 1 mg DRSP/1 mg E2.

It will be understood, of course, that the specific dose level and frequency of dosage for any particular patient will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Because the method of this invention involves effecting HRT, necessarily the individual doses of estrogen and DRSP are administered over a prolonged period of time, *i.e.,* more than one month, usually at least several months and ordinarily for one or more years and often for one or more decades. During that period of time the size of the individual dose of either the estrogen or the DRSP or both can be changed at least once and often two or more times, usually stepwise increased in the case of the estrogen until the minimum effective therapeutic dosage is found. Often, it may be decreased again as the patient progresses from peri to post-menopause, because the estrogen dosage to prevent menopausal bone loss is usually higher than the dosage that is needed for effectively treating climacteric complaints.

Compositions of the invention can be formulated according to accepted pharmaceutical practice, with a conventional pharmaceutically acceptable vehicle, carrier, excipient, binder, preservative, stabilizer, flavor, and/or adjuvant, etc, for any given type of unit dosage form.

Formulations for oral administration are conventional in the art. For example, tablets generally contain a pharmaceutically acceptable carrier, *e.g.*, a binder such as gum tragacanth, acacia, corn starch or gelatin; an excipient such as dicalcium phosphate or celluose; a disintegrating agent such as corn starch or alginic acid; a lubricant, such as magnesium stearate; and/or a sweetening agent or flavoring agent. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup or elixir may contain the active compound, water, alcohol or the like as the carrier, glycerol as solubilized, sucrose as sweetening agent, methyl and propyl parabens as preservatives, a dye and a flavoring such as cherry or orange. When administered orally as a suspension, these compositions may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, disintegrants, diluents and lubricants known in the art.

Formulations suitable for injectable use include sterile aqueous solutions or dispersions and sterile powders for the extemporaneous preparation of sterile injectable solutions or dispersions. The solutions are stable and preserved against the contaminating action of microorganisms such as bacteria and fungi. The injectable solutions or suspensions may be formulated according to known art, using suitable non-toxic, parenterally-acceptable diluents or solvents, such as mannitol, 1,3 butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non irritating excipient, such as cocoa butter, synthetic glyceride esters or polyethylene glycols, which are solid at ordinary temperatures, but liquefy and/or dissolve in the rectal cavity to release the drug.

Methods of formulating HRT compositions for local application (*e.g.*, as extrudable viscous liquids, semi-solid preparations such as gels, ointments or creams, or a spreadable solid such as a stick deodorant), and of applying them to a patient, *e.g.*, to a surface such as skin or mucosa, are disclosed in USP 6,083,528.

In the foregoing and in the following examples, all temperatures are set forth uncorrected in degrees Celsius; and, unless otherwise indicated, all parts and percentages are by weight.

### BRIEF DESCRIPTION OF THE FIGURES

Figure 1 - shows the mean change from baseline (pretreatment day 1) for 24 hour systolic blood pressure on treatment day 14.
Figure 2 - shows the mean change from baseline (pretreatment day 1) for 24 hour diastolic blood pressure on treatment day 14.
Figure 3 - shows changes in systolic 24 hour ambulatory blood pressure from baseline (pretreatment day 1) at steady state (day 14) in subjects (mildly hypertensive postmenopausal women maintained on enalapril maleate 20 mg/day for the duration of treatment) receiving DRSP/E2 or placebo. Horizontal lines represent means.
Figure 4 - shows changes in diastolic 24 hour ambulatory blood pressure from baseline (pretreatment day 1) at steady state (day 14) in subjects (mildly hypertensive postmenopausal women maintained on enalapril maleate 20 mg/day for the duration of treatment) receiving DRSP/E2 or placebo. Horizontal lines represent means.
Figure 5 - shows daytime and nighttime change in systolic 24 hour ambulatory blood pressure from baseline (pretreatment day 1) at steady state (day 14) in subjects (mildly hypertensive postmenopausal women maintained on elanapril maleate 20 mg/day for the duration of treatment) receiving DRSP/E2 or placebo. Horizontal lines represent means.
Figure 6 - shows daytime and nighttime change in diastolic 24 hour ambulatory blood pressure from baseline (pretreatment day 1) at steady state (day 14) in subjects (mildly hypertensive postmenopausal women maintained on elanapril maleate 20 mg/day for the duration of treatment) receiving DRSP/E2 or placebo. Horizontal lines represent means.
Figure 7 - shows mean systolic blood pressure across study days (office blood pressure, except days 1 and 14 are mean 24 ambulatory blood pressure). Study day 1 refers to pretreatment day 1.
Figure 8 - shows mean diastolic blood pressure across study days (office blood pressure, except days 1 and 14 are mean 24 ambulatory blood pressure). Study day 1 refers to pretreatment day 1.

### EXAMPLES

### Example I Administration of DRSP /E2 to mildly hypertensive postmenopausal women being treated with an ACE inhibitor - methods

This study was a double-blind, randomized, 2-parallel group study to compare the effects of DRSP 3 mg/E2 1 mg versus placebo on 24-hour ambulatory blood pressure in postmenopausal mildly hypertensive subjects treated with (e.g., maintained on) enalapril maleate 10 mg twice daily.

### Subjects

To be included in the study each subject had to meet the following criteria: healthy, postmenopausal (natural or surgical), 45-75 years of age who were under treatment with ACE inhibitor enalapril maleate (10 mg bid daily dose for a minimum of 7 days) for mild hypertension and who weighed not less than 100 pounds and who were within ± 15% of normal according to subject's height and frame size. Subjects had systolic blood pressure equal to or less than 150 mm Hg and diastolic blood pressure equal to or less than 90 mm Hg. Subjects must have had at screening a serum potassium between 3.5 and 4.5 mEq/L and screening creatinine clearance greater than 60 mL/min. Subjects were excluded from the study if, during screening evaluations, any of the following criteria applied: clinically significant abnormal laboratory values and electrocardiogram (ECG) abnormalities; significant history of disease, history of drug or alcohol abuse in the past 10 years, positive tests of hepatitis B or C and HIV; previous diagnosis of cancer of any type; smokers in the previous one year; subjects who donated a pint or more of blood within 3 months of the study. a known sensitivity to estrogen therapy, DRSP or enalapril.

### Study Design

This was a randomized, double-blind, 2-parallel groups study. The treatments were:

| | |
|---|---|
| Test | Enalapril maleate 10 mg oral tablet twice daily (bid) + DRSP (3 mg)/E2 (1 mg) oral tablet daily for 14 days |
| Placebo | Enalapril maleate 10 mg oral tablet bid + DRSP/E2 look-alike placebo oral tablet daily for 14 days |

The subjects began to take enalapril at least 7 days prior to the treatment period and continued throughout the treatment period (14 days). Accordingly, 24 postmenopausal women were randomly assigned to 1 of the above treatments. The study was conducted primarily on an out patient basis. On Pretreatment Day 1, subjects entered the clinic for 24-hour ambulatory blood pressure and laboratory assessments. On Treatment Day 1 subjects were discharged from the clinic after dosing, which began the 14-day treatment phase consisting of active or placebo treatment, while continuing on enalapril therapy. Subjects reported back to the clinic on Treatment Day 2 and every 2 days after (up to Treatment Day 12) for safety laboratory assessments and plasma concentrations of DRSP. On Treatment Day 14, subjects reported back to the clinic for approximately a 24-hour period during which the last dose of study drug was administered and 24-hour ambulatory blood pressure and laboratory assessments.

### Ambulatory Blood Pressure Monitoring

Ambulatory blood pressure monitoring (ABPM) was performed using a Spacelabs Inc. Model 90207 (Spacelabs Inc., Redmond Washington) on study Pretreatment Day 1 and Treatment Day 14 according to a standard protocol. The monitor was initialized for each patient and the blood pressure cuff placed on the non-dominant arm. Five correlation readings using a mercury sphygmomanometer (Baumanometer, W.A. Baum Co., Inc., Copiague, New York) were obtained to verify the calibration of the monitor. Readings were recorded every 20 minutes during the period 7 AM to 11 PM and then every 30 minutes from 11 PM to 7 AM. After 24-hours the blood pressure monitoring was terminated and the monitor removed. The ABPM data was then transmitted to Biomedical Systems for analysis via a modem connected to a dedicated phone line. Subjects were instructed to keep their arm immobile during the recording of the blood pressure. Showering and strenuous exercise were not allowed.

### Laboratory Assessments

Plasma Renin Activity (PRA) was measured by measuring the generation of Angiotensin I from angiotensinogen by a commercially available RIA. Blood was collected 24 hours prior to dosing (time zero, Pretreatment Day 1) at least 1 hour after rising in the morning at approximately the same time on Pretreatment Day 1 and Treatment Day 14 under fasting conditions. In addition, subjects were to be ambulatory for 30 minutes prior to specimen collection. Subjects were to refrain from taking medications, preferably 3 weeks prior to blood collection.

Serum aldosterone was measured by a commercially available RIA. Blood was collected 24 hours prior to dosing (time zero, Pretreatment Day 1). Subjects were in the ambulatory position for 2 hours before and during blood and refrained from taking medications, preferably 3 weeks prior to blood collection.

### Statistical Methods

The 24-hour mean systolic and diastolic blood pressure data were analyzed with an ANCOVA using baseline values (Pretreatment Day 1) as covariates. It was assumed that the blood pressure data were normally distributed with common variances. The sources of variation were the: treatments (fixed factor with the levels test and placebo), and the baseline value (covariate). The goodness of fit was checked using a residual plot. The hypothesis tested was H∘: µ_{T} = µ_{P} versus the alternate H₁: µ_{T} ≠ µ_{P} at the significance level of 5%. The individual change from baseline of both systolic and diastolic 24-hour, daytime and nighttime blood pressure data were analyzed using paired-t-test.

### Example II Administration of DRSP /E2 to mildly hypertensive postmenopausal women being treated with an ACE inhibitor - results

### Demographic Data

A total of 24 mildly hypertensive but otherwise healthy postmenopausal women were randomized for this double-blind, two-parallel groups study. The subjects were postmenopausal women with a mean (± standard deviation [SD] ) age of 57 ± 5,62 ± 6 years, and weight of 77 ± 11,70 ± 7 kg, for the placebo and DRSP/E2 treatments, respectively. The study population comprised Caucasian (4%), African American (4%) and Hispanic (92%). All twenty-four subjects completed the study.

### 24-hour ambulatory blood pressure and heart rate

Table 1 presents a summary of 24-hour ambulatory blood pressure data for both the placebo and DRSP/E2 groups.

**Table 1: 24-Hour Ambulatory Blood Pressure Assessment (mm Hg). Data is Mean (SD).**

| Pretreatment Day 1 | | | | Treatment Day 14 | | | |
|---|---|---|---|---|---|---|---|
| Placebo + ACE Inhibitor (n=12) | | DRSP/E2 + ACE Inhibitor (n=12) | | Placebo + ACE Inhibitor (n=12) | | DRSP/E2 + ACE Inhibitor (n=12) | |
| Systolic | Diastolic | Systolic | Diastolic | Systolic | Diastolic | Systolic | Diastolic |
| 139 (12) | 83(8) | 139 (19) | 80(7) | 139 (11) | 83(9) | 130* (16) | 75** (5) |

| | | | | | | | |
|---|---|---|---|---|---|---|---|
| ACE = angiotensin converting enzyme; DRSP = drospirenone; E2 = estradiol ^{*}p=0.006 Treatment Day 14 baseline corrected mean DRSP/E2 versus placebo ^{**}p=0.003 Treatment Day 14 baseline corrected mean DRSP/E2 versus placebo | | | | | | | |

Both systolic and diastolic blood pressures were significantly lower in the DRSP/E2 group relative to those of the placebo group. The baseline adjusted declines were 9 mm Hg (p=0.006) for systolic and 6 mm Hg (p=).003) for the diastolic. See Figures 1 and 2. The changes in systolic and diastolic blood pressure from baseline for the individual subjects are presented in Figures 3 and 4. There was no significant change in either systolic or diastolic blood pressure observed in the placebo group from Pretreatment Day 1 (baseline) to Treatment Day 14. For the DRSP/E2 group, the mean systolic/diastolic blood pressure declined from 139/80 mm Hg at baseline to 130/75 mm Hg on Treatment Day 14. A statistically significant difference between DRSP/E2 and placebo treatment groups was observed with respect to the change from baseline in regard to both systolic (p=0.014) and diastolic (p=0.007) blood pressures.

Changes from baseline in daytime and nighttime systolic and diastolic blood pressure for the placebo and DRSP/E2 groups are presented in Figures 5 and 6. There was no significant change from baseline in either systolic or diastolic blood pressure for the placebo group during both daytime and nighttime blood pressure monitoring. However, in the DRSP/E2 group, there was a significant decrease in daytime systolic (p=0.007) and diastolic (p=0.006) blood pressures from baseline. The nighttime blood pressure did not have a significant change.

Mean changes in hourly blood pressure from baseline on Day 14 for 24-hour ambulatory systolic and diastolic pressures are presented in Figures 7 and 8. The mean change from baseline for subjects in the placebo treatment group fluctuated about the zero line suggesting no trend during the 24-hour monitoring period. On the other hand, for subjects in the DRSP/E2 group, the mean change from baseline was generally below the zero line suggesting a sustained and consistent blood pressure lowering effect.

Twenty-four hour, daytime and nighttime heart rate changes from Pretreatment Day 1 to Treatment Day 14 did not differ between the placebo and DRSP/E2 treatment groups.

### Serum and plasma pharmacodynamic variables

The changes in PRA and serum aldosterone from Pretreatment Day 1 to Treatment Day 14 demonstrated considerable variability among the subjects. The change in PRA (mean (SD)) was 1.13 (2.12) ng/mL/h for the DRSP/E2 group and did not differ from that of the placebo group 2.3 (5.1) ng/mL/h; p=0.50. On the other hand, the serum aldosterone increased by 2.6 (4.5) ng/dL in the DRSP/E2 group, and fell by 0.3 (5.5) ng/dL in the placebo group, a difference which approached significance (p=0.08). In DRSP/E2 treated subjects, the observed increases in serum aldosterone relative to baseline are consistent with a mild antimineralocorticoid effect due to DRSP. DRSP pharmacokinetic data and safety serum assessments of potassium are reported in Karara et al. (2001), J. Clin. Pharmacol. 41, 1014-1033.

### Example III Administration of DRSP/E2 to hypertensive postmenopausal women not being treated with an ACE inhibitor.

In a multicenter, double-blind randomized study examining the effect on the endometrium of 1142 postmenopausal women treated for 1 year with 1 of 4 doses of DRSP (0.5,1,2, and 3 mg) compared with E2 alone, mean systolic and diastolic blood pressure decreased from baseline for all treatment groups. In a *post hoc* analysis of a subset of women (E2 1 mg N= 15, E2 1 mg + 0.5 mg DRSP N=20, E2 1 mg + 1 mg DRSP N=27, E2 1 mg + 2mg DRSP N=15, E2 1 mg + 3 mg DRSP N=25) with elevated BP at baseline (systolic BP>=140mm Hg and or diastolic BP>=90mm Hg, mean systolic and diastolic blood pressures decreased in all groups after 11 weeks although a greater mean decrease was observed in systolic values. The mean decrease in systolic blood pressure was greater for the DRSP + E2 groups (-8.9 mm Hg to -12.7) compared with the E2 alone group (-3.7 mm Hg). This mean decrease was statistically significant (p<0.05) in all DRSP + E2 groups.

From the foregoing description, one skilled in the art can easily ascertain the essential characteristics of this invention, and without departing from the spirit and scope thereof, can make changes and modifications of the invention to adapt it to various usage and conditions.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

The entire disclosure of all applications, patents and publications, cited above and in the figures are hereby incorporated in their entirety by reference.

Aspects of the invention include a method to lower blood pressure and effect hormone replacement therapy (HRT) in a hypertensive human female mammal (woman) receiving HRT, comprising administering to said woman effective amounts of drospirenone (DRSP) and an estrogen; wherein said combination is administered orally, transdermally or by injection; wherein said estrogen is ethinyl estradiol, 17β-estradiol, or a conjugated estrogen; wherein the estrogen is 17β-estradiol at 1-3 mg/day, and the drospirenone is at 1-3 mg/day; and/or wherein the women is post-menopausal.

Another aspect is a method to lower blood pressure and effect hormone replacement therapy (HRT) in a hypertensive human female (woman) receiving HRT, wherein said woman is taking antihypertensive medication, comprising administering to said woman effective amounts of drospirenone (DRSP) and an estrogen; wherein the woman begins taking the hypertensive medication before, or after, said combination is administered; wherein the woman begins taking the hypertensive medication in conjunction with the administration of said combination; wherein said antihypertensive medication is a diuretic, an α-adrenergic blocking agent, an adrenergic neuron blocking agent, a vasodilator, an angiotensin I converting enzyme (ACE) inhibitor, a calcium channel blocker, or a renin inhibitor; wherein said antihypertensive medication is an ACE inhibitor; wherein said combination is administered orally, transdermally or by injection; wherein said estrogen is ethinyl estradiol, 17β-estradiol, or a conjugated estrogen; wherein the estrogen is 17β-estradiol at 1-3 mg/day, and the drospirenone is at 1-3 mg/day; and/or wherein the woman is post-menopausal.

In another aspect, the blood pressure of hypertensive women administered the combination of drospirenone and estradiol without an antihypertensive medication was also unexpectedly reduced, see Example 3.

## Claims

1. The use of drospirenone (DRSP) and 17β-estradiol for the manufacture of a medicament for lowering blood pressure and effecting hormone replacement therapy (HRT) in a hypertensive human female patient, wherein said medicament is adapted for administration of DRSP in an amount of 0.5-3 mg/day and 17β-estradiol in an amount of 1,0 - 3 mg/day.

2. The use of claim 1, wherein said medicament is adapted to be administered orally, transdermally or by injection.

3. The use of claim 2, wherein said medicament is adapted to be administered orally.

4. The use of claim 1, wherein the patient is perimenopausal, menopausal or post-menopausal.

5. The use of claim 1, wherein said hypertension is mild or severe.

6. The use of claim 1, wherein said amount of DRSP is 3 mg and said amount of 17β-estradiol is 1 mg.

7. The use of claim 1, wherein said amount of DRSP is 2 mg and said amount of 17β-estradiol is 1 mg.

8. The use of claim 1, wherein said amount of DRSP is 1 mg and said amount of 17β-estradiol is 1 mg.
